# EUROPÄISCHE PATENTSCHRIFT

(11) **EP 1 834 658 B1**
(45) Veröffentlichungstag und Bekanntmachung des Hinweises auf die Patenterteilung: **30.12.2009**
(21) Anmeldenummer: 06111091.2
(22) Anmeldetag: 14.03.2006
(51) Int. Cl.: A61M 5/142

(54) **Peristaltische Mikropumpe mit Volumenstromsensor**
Peristaltic micropump with volume flow sensor
Micropompe péristaltique avec capteur de débit

(43) Veröffentlichungstag der Anmeldung: 19.09.2007
(73) Patentinhaber: F. Hoffmann-La Roche AG, 4070 Basel (CH); Roche Diagnostics GmbH, 68305 Mannheim (DE)
(72) Erfinder: Haar, Hans-Peter, Dr., 69168 Wiesloch (DE)
(74) Vertreter: Dick, Alexander

(56) Entgegenhaltungen:
- US-A- 5 049 047
- US-A- 5 205 819
- US-A- 5 478 211
- US-A1- 2002 071 785
- US-A1- 2004 204 673
- US-A1- 2005 075 624
- US-B1- 6 349 740

## Beschreibung

Die Erfindung betrifft eine Mikropumpe zum peristaltischen Pumpen eines flüssigen Mediums und eine Vorrichtung zur dosierten Verabreichung eines flüssigen Medikaments, die eine peristaltische Mikropumpe enthält.

Für die Verabreichung von flüssigen Medikamenten kommen unter anderem tragbare Infusionsgeräte zum Einsatz. Diese Infusionsgeräte ersetzen Injektionen mit einer Spritze oder einem Injektions-Pen.

Ein Beispiel für ein solches Infusionsgerät ist eine Insulinpumpe zur Verabreichung von Insulin, die insbesondere von Diabetikern, die mit einer intensivierten Therapie behandelt werden und regelmäßig ihre Blutglukosewerte messen, verwendet wird. Die Insulinpumpe wird vom Patienten außen am Körper, zum Beispiel am Gürtel getragen. Über einen dünnen Schlauch (Infusions-Set oder Katheter), dessen Kanüle unter der Haut des Patienten sitzt, gibt die Infusionspumpe kontinuierlich Insulin zur Deckung des Insulingrundbedarfs an den Körper des Patienten ab. Das zum Beispiel zu Mahlzeiten benötigte zusätzliche Insulin kann per Knopfdruck durch den Patienten abgerufen werden.

Im Stand der Technik bekannte Infusionsgeräte enthalten eine Pumpe, mit deren Hilfe das zu verabreichende flüssige Medikament aus dem Infusionsgerät gepumpt wird. Üblicherweise enthält das Infusionsgerät ein Behältnis als Reservoir mit flüssigem Medikament, aus dem das Medikament mittels eines Kolbens verdrängt und verabreicht wird. Ein Infusionsgerät mit einer solchen, einen Verdrängerkolben umfassenden Pumpe ist beispielsweise aus WO 98/47552 A1 oder aus WO 01/24854 A1 bekannt.

Als Alternative zu Pumpen mit Verdrängerkolben sind im Stand der Technik peristaltische Pumpen bekannt. US 2005/0123420 A1 bezieht sich auf eine peristaltische Mikropumpe mit drei Membranbereichen, die je einen Piezoaktor zum Betätigen des jeweiligen Membranbereichs aufweisen. Ein Pumpkörper bildet mit dem ersten und dritten Membranbereich zwei Ventile, deren Durchlassöffnung im unbetätigsten Zustand des zugehörigen Membranbereichs offen und durch Betätigen des Membranbereichs verschließbar ist. Der Pumpenkörper bildet mit dem zweiten Membranbereich eine Pumpkammer, deren Volumen durch Betätigen des zweiten Membranbereichs verringerbar ist. Das erste und das zweite Ventil sind mit der Pumpkammer fluidmäßig verbunden. Weitere mit piezoelektrischen Aktoren arbeitende Mikropumpen sind zum Beispiel aus EP 0 949 418 B1 und US 6,416,294 B1 bekannt.

Das Dosieren geringster Flüssigkeitsmengen im Mikro- bis Nanoliterbereich wird neben der Medizintechnik für viele Anwendungsbereiche, zum Beispiel für die Analytik oder die Umwelttechnik, immer relevanter. Daher müssen Pumpenanordnungen gefunden werden, bei denen automatisch überwacht wird, ob die gewünschte Flüssigkeitsmenge tatsächlich abgegeben wurde. Eine Abweichung der abgegebenen Menge von der gewünschten Menge kann beispielsweise durch eine Fehlfunktion der Pumpe oder eine Okklusion der Pumpwege verursacht werden.

Peristaltische Mikropumpen gemäß dem Oberbegriff des Anspruchs 1 sind aus der US-A-5 205 819 und der US-A-5 049 047 bekannt.

Aufgabe der vorliegenden Erfindung ist es , die Nachteile des Standes der Technik zu vermeiden und insbesondere eine Überwachung der durch eine Mikropumpe abgegebenen Flüssigkeitsmenge zu ermöglichen. Ferner ist es eine Aufgabe der vorliegenden Erfindung, eine peristaltische Mikropumpe mit einer erhöhten Betriebssicherheit bereitzustellen.

Diese Aufgabe wird erfindungsgemäß gelöst durch eine Mikropumpe zum peristaltischen Pumpen eines flüssigen Mediums, gemäß Anspruch 1.

Die erfindungsgemäße Pumpe umfasst mindestens einen Fließkanal mit einem Wandkörper. Der Wandkörper ist zum Beispiel ein Schlauch, insbesondere ein Infusionsschlauch oder er umfasst mindestens einen Membranbereich. Zumindest in Teilabschnitten des Fließkanals ist seine Querschnittsfläche durch Krafteinwirkung auf den Wandkörper veränderbar. Vorzugsweise ist der Wandkörper zumindest in diesen Teilabschnitten aus einem elastischen Material gefertigt. Ferner umfasst die erfindungsgemäße Pumpe Aktoren die in einem Kanalabschnitt des Fließkanals auf den Wandkörper eine Kraft ausüben und dadurch die Querschnittsfläche in dem Kanalabschnitt verringern können. Somit kann ein in diesem Kanalabschnitt enthaltenes flüssiges Medium zumindest teilweise aus dem Kanalabschnitt verdrängt werden. Mittels der erfindungsgemäßen Mikropumpe können in Abhängigkeit von der Gestaltung des Wandkörpers, des Fließkanals und der Aktoren kleine Mengen an flüssigem Medium, insbesondere herunter bis zu 10 nL/min gefördert werden. Dies wird insbesondere dadurch möglich, dass im Unterschied zu den beschriebenen Infusionspumpen mit einem Verdrängerkolben in dem Reservoir von flüssigem Medium bei der erfindungsgemäßen Mikropumpe kleine Volumina aus kleinen Volumina heraus gefördert werden.

Die peristaltische Mikropumpe der vorliegenden Erfindung umfasst ferner eine Sensoranordnung zur Detektion einer Menge des flüssigen Mediums in dem Kanalabschnitt, dessen Querschnittsfläche durch Krafteinwirkung veränderbar ist. Diese Sensoranordnung ist außerhalb des Fließkanals angeordnet. Sie detektiert daher die Menge des flüssigen Mediums in dem Kanalabschnitt ohne eine direkte Berührung des flüssigen Mediums.

Die erfindungsgemäße peristaltische Mikropumpe umfasst ferner mindestens zwei weitere Sensoranordnungen zur Detektion der Menge des flüssigen Mediums in den mindestens zwei verschließbaren Kanalabschnitten, wobei die mindestens zwei weiteren Sensoranordnungen außerhalb des Fließkanals angeordnet sind.

Weiterhin wird ein Verfahren zur Überwachung einer Mikropumpe zum peristaltischen Pumpen eines flüssigen Mediums beschrieben, wobei die Mikropumpe
A) mindestens einen Fließkanal mit einem Wandkörper umfasst, wobei der Fließkanal eine Querschnittsfläche aufweist, die zumindest in Teilabschnitten des Fließkanals durch Krafteinwirkung auf den Wandkörper des Fließkanals veränderbar ist und
B) mindestens einen Aktor umfasst, der so angeordnet ist, dass er auf den Wandkörper des Fließkanals in einem Kanalabschnitt eine Kraft zum Verringern der Querschnittsfläche in dem Kanalabschnitt ausüben kann.

Bei dem nicht beanspruchten Verfahren detektiert mindestens eine außerhalb des Fließkanals angeordnete Sensoranordnung eine Menge des flüssigen Mediums in dem Kanalabschnitt. Die Sensoranordnung detektiert bei der vorliegenden Erfindung ein Messsignal (z.B. Absorption, Fluoreszenz, Kapazität) welches unter Kenntnis anderer Parameter (z.B. Fließkanaldurchmesser, Länge des Kanalabschnitts, Eigenschaften des flüssigen Mediums) z.B. die Berechnung eines Volumens des flüssigen Mediums in dem Kanalabschnitt ermöglicht.

Durch Messung der Menge des flüssigen Mediums mit der Sensoranordnung in der erfindungsgemäßen Mikropumpe kann sichergestellt werden, dass das flüssige Medium in der angestrebten Menge durch die Pumpe abgegeben wird bzw. dass eine Fehlabgabe erkannt wird. Dadurch dass mehrere Sensoranordnungen vorgesehen sind, lässt sich die Menge an flüssigem Medium sogar in einzelnen Bereichen der Pumpe messen und verfolgen. Damit wird unabhängig von der Pumpenmechanik gewährleistet, dass eine Überwachung stattfindet, ob die Mikropumpe wunschgemäß arbeitet und tatsächlich flüssiges Medium in der richtigen Menge abgibt. Eine solche Überwachung ist insbesondere bei Infusionspumpen für flüssige Medikamente wichtig, zum Beispiel für Insulinpumpen, da eine falsche Dosierung zu gesundheitlichen Schäden führen kann.

Gemäß der vorliegenden Erfindung umfasst die Mikropumpe mindestens zwei Aktoren, die so angeordnet sind, dass sie auf den Wandkörper des Fließkanals in mindestens zwei Kanalabschnitten eine Kraft zum vorübergehenden Verschließen des jeweiligen Kanalabschnitts ausüben können, so dass ein Volumensegment in dem Fließkanal eingrenzbar ist, wobei eine Sensoranordnung außerhalb des Wandkörpers des Fließkanals zur Detektion der Menge des flüssigen Mediums in dem Volumensegment dient. Bei dem vorgestellten Verfahren verschließen mindestens zwei Aktoren vorübergehend zwei Kanalabschnitte und schließen dabei zwischen den zwei verschließbaren Kanalabschnitten ein Volumensegment ein, wobei in dem Volumensegment weiter zu pumpendes flüssiges Medium enthalten sein kann. Eine außerhalb des Fließkanals angeordnete Sensoranordnung detektiert die Menge des flüssigen Mediums in dem Volumensegment.

Eine erste Variante dieser Ausführungsform ist zum Beispiel eine Mikropumpe mit mehreren rollenförmigen Aktoren, die auf einen kreisförmigen Umfang um eine rotierende Welle angeordnet sind (in einer Rotationspumpe), wobei der Fließkanal bogenförmig in einem Bereich entlang des kreisförmigen Umfangs verläuft. Je zwei rollenförmige Aktoren können dann den Fließkanal in denjenigen Kanalabschnitten vorübergehend durch Krafteinwirkung von außen verschließen, an die sie aktuell grenzen. Dies erfolgt dadurch, dass die rollenförmigen Aktoren den Wandkörper an der Stelle, an der sie sich jeweils befinden, zusammendrücken und dadurch die Querschnittsfläche des Fließkanals in dem jeweiligen Kanalabschnitt nahezu auf Null reduzieren. Zwischen je zwei durch die Kraft der rollenförmigen Aktoren verschlossenen Kanalabschnitten befindet sich ein Volumensegment in dem Fließkanal, das zum Beispiel flüssiges Medium oder Luft enthalten kann. Beim Rotieren der Welle der Pumpe bewegen sich die rollenförmigen Aktoren entlang des bogenförmigen Fließkanals, so dass sich die mittels der Aktoren verschlossenen Kanalabschnitte und damit auch das in dem dazwischen liegenden Kanalabschnitt liegende Volumensegment ebenfalls entlang des Fließkanals verschieben. Außerhalb des Fließkanals ist mindestens eine Sensoranordnung angeordnet, die in einem Volumensegment, das durch die Sensoranordnung erfassbar ist, die Menge des flüssigen Medikaments in dem Volumensegment detektiert.

Diese erste Variante betrifft daher eine Mikropumpe, die eine Rotationspumpe mit rollenförmigen Aktoren ist, wobei die rollenförmigen Aktoren kreisförmig um eine rotierbare Welle angeordnet sind und der Fließkanal bogenförmig entlang eines an die rollenförmigen Aktoren angrenzenden Bereichs verläuft. Diese Rotationspumpe fördert die gewünschte Menge an flüssigem Medium durch das Verschieben kleiner definierter Volumensegmente mittels der rollenförmigen Aktoren, wie bereits beschrieben.

Eine zweite Variante dieser Ausführungsform ist zum Beispiel eine Mikropumpe, die drei stempelförmige Aktoren umfasst, die nebeneinander an dem Wandkörper des Fließkanals angeordnet sind. Die zwei äußeren Aktoren (Ventilstempel) können dann vorübergehend den Fließkanal durch Krafteinwirkung verschließen, so dass in dem Kanalabschnitt zwischen den durch die zwei Aktoren verschlossenen Kanalabschnitten ein Volumensegment eingegrenzt wird. Eine diesem mittleren Kanalabschnitt zugeordnete Sensoranordnung detektiert eine in dem Volumensegment enthaltene Menge an flüssigem Medium. Diese Menge kann auch Null betragen, zum Beispiel falls der mittlere Aktor (Pumpenstempel) den mittleren Kanalabschnitt ebenfalls verschließt oder falls in dem Kanalabschnitt Luft und kein flüssiges Medium enthalten ist.

Diese zweite Variante betrifft daher eine Mikropumpe, die mindestens drei nebeneinander an dem Wandkörper des Fließkanals angeordnete stempelförmige Aktoren enthält, die mindestens zwei Ventilstempel und mindestens einen Pumpenstempel umfassen. Diese nach dem peristaltischen Prinzip arbeitende Pumpe fördert die gewünschte Menge an flüssigem Medium durch das gezielte Öffnen und Schließen der drei den drei stempelförmigen Aktoren zugeordneten, nebeneinander liegenden Kanalabschnitte in einer bestimmten Reihenfolge, so dass die gewünschte Menge an flüssigem Medium in einer bestimmten Richtung in dem Fließkanal weiterbewegt wird.

Gemäß der vorliegenden Erfindung enthält die Mikropumpe mindestens zwei weitere Sensoranordnungen zur Detektion der Menge des flüssigen Mediums in den mindestens zwei verschließbaren Kanalabschnitten, wobei die mindestens zwei weiteren Sensoranordnungen außerhalb des Fließkanals angeordnet sind. Die zwei weiteren außerhalb des Fließkanals angeordneten Sensoranordnungen detektieren die Menge des flüssigen Mediums in den zwei durch die Ventilstempel verschließbaren Kanalabschnitten. Dadurch kann detektiert werden, welche Menge an flüssigem Medium sich aktuell in dem jeweiligen Kanalabschnitt befindet und somit die Bewegung des flüssigen Mediums durch die Kanalabschnitte verfolgt werden.

Die Aktoren der erfindungsgemäßen Mikropumpe können zum Beispiel einen Piezoaktuator, einen Bimetallaktuator oder einen Exzenter an einer rotierenden Welle umfassen. Eine Mikropumpe mit drei an dem Wandkörper des Fließkanals angeordneten stempelförmigen Aktoren umfasst zum Beispiel drei Piezoaktoren, drei Bimetallaktuatoren oder drei Exzenter an einer rotierenden Welle. Ferner können auf Formgedächtnislegierungen (shape memory alloys) basierende Aktoren oder Magnetpulsschalter mit zwei Zuständen als Aktoren der erfindungsgemäßen Mikropumpe zum Einsatz kommen. Die Aktoren reagieren auf gesteuerte Änderungen bestimmter Größen wie Temperatur oder elektrische Spannung mit definierten Aktorwirkungen, durch die gezielt eine Kraft auf den Wandkörper des Fließkanals zum Verringern der Querschnittsfläche eines Kanalabschnitts ausgeübt wird oder nicht.

Gemäß einer weiteren Ausführungsform der vorliegenden Erfindung dient als Aktor eine rotierbare Scheibe, die um eine exzentrische Achse rotiert, wobei der Fließkanal annähernd ringförmig in einem an die rotierbare Scheibe oder an eine durch die rotierbare Scheibe betätigbare weitere Scheibe angrenzenden Bereich verläuft.

Gemäß einer bevorzugten Ausführungsform der vorliegenden Erfindung umfasst die Sensoranordnung der Mikropumpe, ein elektromagnetische Strahlung emittierendes Element, das auf einen zumindest teilweise strahlungsdurchlässigen Bereich des Wandkörpers des Fließkanals gerichtet ist, und ein elektromagnetische Strahlung detektierendes Element, das ebenfalls auf einen zumindest teilweise strahlungsdurchlässigen Bereich des Wandkörpers des Fließkanals gerichtet ist und das so angeordnet ist, dass es aus dem Bereich des Wandkörpers austretende elektromagnetische Strahlung, die den Fließkanal durchquert hat, detektieren kann. Das Messverfahren dieser Sensoranordnung beruht auf der Absorption der elektromagnetischen Strahlung oder der Erzeugung von Fluoreszenz in dem Kanalabschnitt, in den das Strahlung emittierende Element elektromagnetische Strahlung einstrahlt. Die Absorption wird durch Messen des nicht absorbierten (transmittierten) Anteils der eingestrahlten elektromagnetischen Strahlung mittels des elektromagnetische Strahlung detektierenden Elements bestimmt. Der absorbierte Anteil hängt dabei (neben dem Material und Strahlungsweg durch den Wandkörper) von den Materialien und dem Strahlungsweg durch die Materialien in dem Kanalabschnitt ab. Wenn daher das Absorptionsverhalten des flüssigen Mediums und die möglichen Abmessungen des Fließweges im Bereich des Kanalabschnitts, an dem die Sensoranordnung angeordnet ist, bekannt sind, kann anhand der gemessenen Intensität der transmittierten Strahlung bzw. anhand der Intensität von durch die emittierte elektromagnetische Strahlung erzeugter Fluoreszenzstrahlung die Menge des flüssigen Mediums in dem Kanalabschnitt bestimmt werden.

Vorzugsweise ist die eingestrahlte elektromagnetische Strahlung monochromatisch und hat eine Wellenlänge, die auf die Absorptionseigenschaften oder gegebenenfalls eine Fluoreszenzwellenlänge des flüssigen Mediums abgestimmt ist. Die mindestens eine Sensoranordnung der erfindungsgemäßen Mikropumpe umfasst dazu ein elektromagnetische Strahlung emittierendes Element, das elektromagnetische Strahlung mit einer Wellenlänge emittiert, die vorzugsweise im Bereich eines Absorptionsmaximums des flüssigen Mediums oder bei einer Fluoreszenzwellenlänge des flüssigen Mediums liegt. Enthält das flüssige Medium zum Beispiel Wasser, so kann eine Wellenlänge der eingestrahlten elektromagnetischen Strahlung im Bereich des Absorptionsmaximums von Wasser bei 3 µm gewählt werden, zum Beispiel eine Wellenlänge zwischen 3 µm und 15 µm. Wenn sich dann Wasser in dem Kanalabschnitt befindet, durch den die elektromagnetische Strahlung hindurchgeht, so wird ein großer Anteil der eingestrahlten elektromagnetischen Strahlung absorbiert. Befindet sich zum Beispiel Luft in dem Kanalabschnitt, so wird deutlich weniger absorbiert. Ist der Kanalabschnitt geschlossen (Querschnittsfläche des Fließweges nahezu Null), so wird annähernd gar keine Strahlung absorbiert, abgesehen von dem von dem Material des Wandkörpers absorbierten Anteil.

Es können aber auch die Absorptionseigenschaften oder die Fluoreszenzeigenschaften eines dem flüssigen Medium (gegebenenfalls nur zu diesem Zweck) zugegebenen Hilfsstoffes berücksichtigt werden, zum Beispiel eines Konservierungsstoffes. Dabei muss die Konzentration des Hilfsstoffes in dem flüssigen Medium ausreichend hoch sein, damit die Absorption bzw. die Fluoreszenz der elektromagnetischen Strahlung detektierbar ist. Der Hilfsstoff kann beispielsweise ein Absorptionsmaximum im nahen UV (zum Beispiel bei 320 nm) aufweisen und das elektromagnetische Strahlung emittierende Element Strahlung in diesem Wellenlängenbereich emittieren.

Das Material des Wandkörpers wird bei der vorliegenden Erfindung vorzugsweise so gewählt, dass es eine möglichst geringe Absorption der elektromagnetischen Strahlung verursacht. Falls die erfindungsgemäße Mikropumpe für die dosierte Verabreichung flüssiger Medikamente verwendet wird, muss das Material des Wandkörpers (zum Beispiel des Infusionsschlauches) biokompatibel sein. Für Schläuche von Insulinpumpen kommt bei der vorliegenden Erfindung vorzugsweise mindestens ein Material ausgewählt aus der Gruppe Ethylen-Vinylacetat-Copolymer (EVA), Polyvinylchlorid (PVC), Polyurethan (PU) und Polyethylen (PE) zum Einsatz. Gegebenenfalls können ein Außenschlauch und ein Innenschlauch mit verschiedenen Materialien verwendet werden.

Gemäß einer Ausführungsform der vorliegenden Erfindung kann der Wandkörper einen Wandbereich aufweisen, der zumindest teilweise reflektierend ausgebildet ist und das elektromagnetische Strahlung detektierende Element so angeordnet sein, dass es von dem elektromagnetische Strahlung emittierenden Element emittierte und von dem Wandbereich reflektierte Strahlung detektieren kann. Der Strahlengang der elektromagnetischen Strahlung verläuft dann zweimal durch den Fließkanal in den Kanalabschnitt, in dem die Menge an flüssigem Medium zu detektieren ist, bevor sie durch das detektierende Element erfasst wird.

Gemäß einer anderen Ausführungsform der erfindungsgemäßen Mikropumpe sind das elektromagnetische Strahlung emittierende Element und das elektromagnetische Strahlung detektierende Element auf gegenüberliegenden Seiten des Fließkanals außerhalb des Wandkörpers angeordnet. Die eingestrahlte elektromagnetische Strahlung geht dann nur in einer Richtung durch den Fließkanal, bevor der transmittierte Anteil detektiert wird.

Als elektromagnetische Strahlung emittierendes Element kann bei der vorliegenden Erfindung ein Laser, insbesondere eine Laserdiode, eine Leuchtdiode oder Leuchtelemente aus elektronischen Polymeren dienen. Das elektromagnetische Strahlung emittierende Element wird vorzugsweise so ausgewählt, dass es sich aufgrund seiner Formgebung vorteilhaft mit dem Wandkörper des Fließkanals (z.B. einem Schlauch) verbinden lässt. Als elektromagnetische Strahlung detektierendes Element kann bei der vorliegenden Erfindung eine Photodiode, ein Phototransistor, ein sonstiger Halbleiterdetektor oder eine einfache CMOS Mehrfachdetektoranlage eingesetzt werden. Das elektromagnetische Strahlung detektierende Element kann in eine elektronische Schaltung integriert sein.

Gemäß einer anderen Ausführungsform der vorliegenden Erfindung umfasst die Sensoranordnung einen Kondensator, der auf zwei Seiten des Kanalabschnitts des Fließkanals außerhalb des Wandkörpers angeordnete Kondensatorplatten enthält. Die Sensoranordnung umfasst ferner eine Messanordnung zur Messung der Kapazität des Kondensators. Mit dieser Sensoranordnung kann kapazitiv die Menge an in dem Kanalabschnitt enthaltenem flüssigem Medium bestimmt werden. Die Kapazität des Kondensators wird mit einer dem Fachmann bekannten Messanordnung gemessen, zum Beispiel durch Verstimmung eines Schwingkreises. Die Kapazität wird dabei durch eine zwischen den Kondensatorplatten vorhandene Materialzusammensetzung und -menge bestimmt. Beispielsweise erhöht das Füllen eines zwischen den Kondensatorplatten verlaufenden Schlauches mit Wasser aufgrund der Dielektrizitätskonstante von Wasser von ca. 90 die Kapazität des Kondensators.

Gemäß einer anderen Ausführungsform der vorliegenden Erfindung umfasst die Sensoranordnung einen Ultraschallsensor, der einen Ultraschallsender und einen Ultraschallempfänger enthält, die auf zwei Seiten des Kanalabschnitts des Fließkanals (z.B. nebeneinander oder gegenüberliegend) außerhalb des Wandkörpers angeordnet sind. Der Ultraschallsender erzeugt z.B. mittels eines piezoelektrischen Materials ein Ultraschallsignal, das durch den Wandkörper in den Fleißkanal gelangt. Der Ultraschallsensor empfängt ein aus dem Fließkanal austretendes Ultraschallsignal, dessen Stärke insbesondere von der in dem Fließkanal reflektierten bzw. von der durch diesen transmittierten Ultraschallenergie abhängt. Diese reflektierte bzw. transmittierte Ultraschallenergie wird durch die akustische Impedanz-Fehlanpassung der verwendeten Materialien, insbesondere des Wandkörpermaterials und des in dem Kanalabschnitt enthaltenen Materials, bestimmt. Die akustische Impedanz-Fehlanpassung liefert die Basis für die Detektion der in dem Kanalabschnitt enthaltenen Menge an flüssigem Medium.

Bei den besonders bevorzugten nicht beanspruchten Verfahren zur Überwachung der erfindungsgemäßen Mikropumpe misst folglich die mindestens eine Sensoranordnung die Absorption oder Transmission von elektromagnetischer Strahlung, die Fluoreszenz, ein reflektiertes oder transmittiertes akustisches Signal oder die Änderung der Kapazität in einem Kanalabschnitt.

Die Erfindung bezieht sich weiterhin auf eine Vorrichtung zur dosierten Verabreichung eines flüssigen Medikaments, enthaltend
i) eine erfindungsgemäße Mikropumpe,
ii) ein Reservoir mit dem flüssigen Medikament und
iii) eine Infusionsnadelanordnung,
wobei das Reservoir mit der Infusionsnadelanordnung über den Fließkanal verbunden ist. Eine solche Vorrichtung ist beispielsweise eine Insulinpumpe, die ein vorzugsweise auswechselbares oder wieder befüllbares Reservoir an Insulin umfasst. Das flüssige Medikament wird mittels der Mikropumpe der erfindungsgemäßen Vorrichtung aus dem Reservoir über den Fließkanal in die Infusionsnadelanordnung gepumpt, um über eine Infusionsnadel dosiert einem Patienten verabreicht zu werden. Mittels der in der Mikropumpe enthaltenen Sensoranordnung werden dabei die gepumpte Menge an flüssigem Medikament und die Funktionsfähigkeit der Mikropumpe überwacht.

Die erfindungsgemäße Vorrichtung zur dosierten Verabreichung des flüssigen Medikaments umfasst vorzugsweise eine Steuereinrichtung, die die Mikropumpe ansteuert. Die Steuereinrichtung kann dabei direkt an der Mikropumpe, zum Beispiel in einem gemeinsamen Gehäuse, oder entfernt von der Mikropumpe angeordnet sein. Beispielsweise kann die Steuereinrichtung in einem ersten Gehäuse angeordnet sein, das ein Patient an einem Gürtel befestigen kann, und die Mikropumpe in der Nähe der Infusionsnadelanordnung montiert sein. Die Kommunikation zwischen Steuereinrichtung und Mikropumpe kann dabei drahtlos oder über eine verbindende Signalleitung erfolgen.

Die Mikropumpe kann bei der erfindungsgemäßen Vorrichtung zum Beispiel in einem gemeinsamen Gehäuse mit dem Reservoir, in einem gemeinsamen Gehäuse mit der Injektionsnadelanordnung oder separat außerhalb des Wandkörpers des Fließkanals zwischen dem Reservoir und der Infusionsnadelanordnung angeordnet sein.

Eine Anordnung der Mikropumpe nahe der Infusionsnadelanordnung (unabhängig von der Position einer Steuereinrichtung) hat den Vorteil, dass die Überwachung der durch die Mikropumpe gepumpten Menge an flüssigem Medikament durch die Sensoranordnung nahe der Infusionsstelle stattfindet und somit mit hoher Sicherheit überwacht wird, ob dort die gewünschte Menge verabreicht wird.

Gemäß einer bevorzugten Ausführungsform der vorliegenden Erfindung leitet eine Steuereinrichtung der Mikropumpe eine Reaktion ein, wenn die mit der mindestens einen Sensoranordnung detektierte Menge des flüssigen Mediums in einem Kanalabschnitt von einer erwarteten Menge um mehr als eine festgelegte Differenz abweicht, wobei die Reaktion mindestens eine Reaktion ausgewählt aus der Gruppe Stoppen der Mikropumpe, Abgeben eines optischen Warnsignals, Abgeben eines haptischen Warnsignals, Abgeben eines akustischen Warnsignals und Abgeben eines Signals, das von einem entfernten Empfänger empfangen wird, um dort ein Warnsignal abzugeben, ist.

Das optische Warnsignal kann beispielsweise eine blinkende Warnleuchte oder ein das Problem beschreibender Text auf einer Anzeige (gegebenenfalls an der Steuereinrichtung) sein. Das haptische Warnsignal kann zum Beispiel das Vibrieren einer am Körper des Patienten tragbaren Einrichtung, gegebenenfalls der Steuereinrichtung, sein. Als akustisches Warnsignal kann zum Beispiel ein von der Steuereinrichtung abgegebener Warnton dienen. Die Steuereinrichtung kann auch einem entfernten Empfänger ein Signal übermitteln, das dort das Abgeben eines optischen, haptischen und/oder akustischen Warnsignals auslöst. Der entfernte Empfänger ist zum Beispiel eine Armbanduhr oder ein Handy, die zum Abgeben des Warnsignals zur Kenntnisnahme durch den Patienten selbst oder durch eine andere Person vorgesehen sein können. Die Kommunikation zwischen Steuereinrichtung und Empfänger kann über eine Signalleitung oder drahtlos erfolgen.

Gemäß einer bevorzugten Ausführungsform der vorliegenden Erfindung ist der Wandkörper des Fließkanals in der Mikropumpe lösbar montiert. Dies hat den Vorteil, dass der Wandkörper (zum Beispiel ein Schlauch) als wegwerfbares Einwegteil vorgesehen sein kann, das zu einem gewünschten Zeitpunkt (gegebenenfalls mit dem entleerten Reservoir des flüssigen Mediums und/oder einer gebrauchten Infusionsnadelanordnung) entsorgt und die Mikropumpe wieder verwendet werden kann.

Weiterhin wird die Verwendung einer erfindungsgemäßen Mikropumpe zum Pumpen von Insulin oder einem Schmerzmittel, die Verwendung mindestens einer Sensoranordnung in einer erfindungsgemäßen Mikropumpe zur Überwachung der Menge an gepumptem flüssigem Medium und/oder die Verwendung mindestens einer Sensoranordnung in einer erfindungsgemäßen Mikropumpe zur Okklusionsdetektion offenbart.

Eine Okklusion kann beispielsweise detektiert werden, wenn ein Aktor der Mikropumpe zu einem bestimmten Zeitpunkt einen Kanalabschnitt des Fließkanals verschließen oder zumindest seine Querschnittsfläche verkleinern sollte, die an dem Kanalabschnitt angeordnete Sensoranordnung jedoch keine oder eine zu geringe Mengenabnahme an flüssigem Medium in dem Kanalabschnitt detektiert. Dies deutet auf eine Blockierung des Fließkanals hin.

Anhand der Zeichnung wird die Erfindung nachstehend näher erläutert.

Es zeigen
- Figuren 1A bis 1D: schematisch die Arbeitsweise einer erfindungsgemäßen Mikropumpe mit stempelförmigen Aktoren,
- Figur 2: schematisch eine erfindungsgemäße Mikropumpe mit rollenförmigen Aktoren,
- Figur 3: schematisch eine erfindungsgemäße Mikropumpe mit scheibenförmigem Aktor,
- Figur 4: schematisch das Messprinzip einer ersten Sensoranordnung zur Messung der Absorption von elektromagnetischer Strahlung in einer erfindungsgemäßen Mikropumpe,
- Figur 5: schematisch eine zweite Sensoranordnung zur Messung der Absorption von elektromagnetischer Strahlung oder der Reflexion von Ultraschall in einer erfindungsgemäßen Mikropumpe,
- Figur 6: schematisch eine Sensoranordnung zur Kapazitätsmessung oder zur Ultraschallmessung in einer erfindungsgemäßen Mikropumpe,
- Figur 7: eine erfindungsgemäße Vorrichtung zur dosierten Verabreichung eines flüssigen Medikaments und

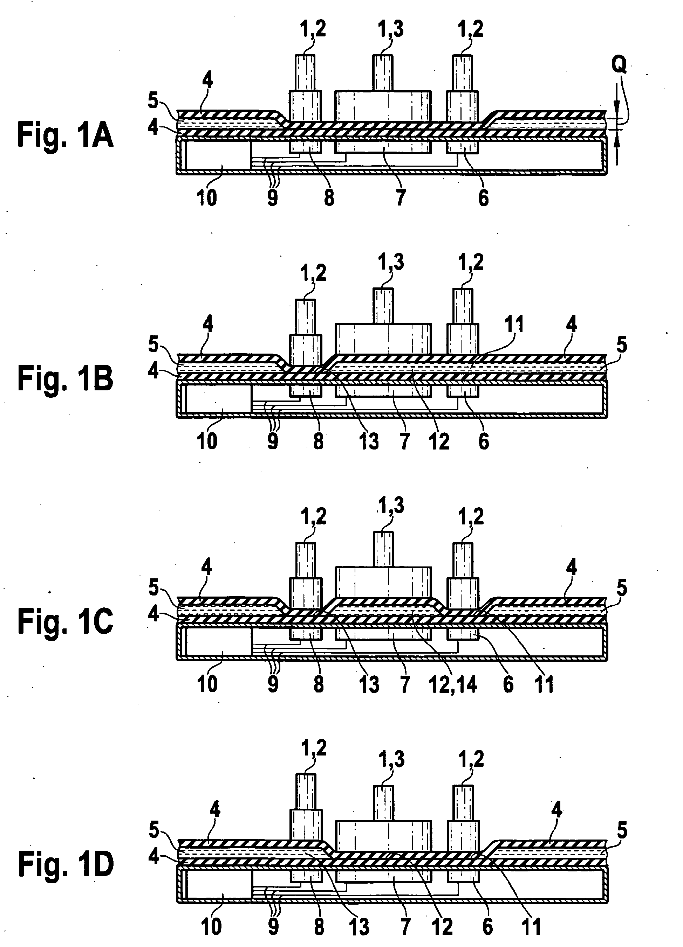

Figuren 1A bis 1D zeigen eine Bewegungssequenz von stempelförmigen Aktoren in einer erfindungsgemäßen Mikropumpe.

Die Mikropumpe weist drei stempelförmige Aktoren 1 auf, zwei Ventilstempel 2 und einen Pumpenstempel 3. Die drei Aktoren 1 sind nebeneinander außen an einem Wandkörper 4 eines Fließkanals 5 angeordnet. Der Wandkörper 4 ist zumindest im Bereich der drei Kanalabschnitte, die von den Aktoren flankiert werden, elastisch ausgebildet, so dass die Aktoren die Querschnittsfläche Q des Fließkanals 5 durch Ausüben einer Kraft auf den Wandkörper des jeweiligen Kanalabschnitts verringern können. Die erfindungsgemäße Mikropumpe weist ferner drei Sensoranordnungen 6, 7, 8 auf, die jeweils einem Kanalabschnitt an jeweils einem stempelförmigen Aktor 1 zugeordnet sind. Die Sensoranordnungen 6, 7, 8 dienen zum Messen der Menge des flüssigen Mediums in dem jeweiligen Kanalabschnitt. Die Sensoranordnungen 6, 7, 8 sind über Signalleitungen 9 mit einer Auswerteeinrichtung 10 zur Auswertung der mit der jeweiligen Sensoranordnung 6, 7, 8 gemessenen Signale verbunden.

Zum Pumpen eines Volumensegments (von rechts nach links in der Figur) durchläuft die erfindungsgemäße Mikropumpe die in den Figuren 1A bis 1D dargestellte Bewegungssequenz. In Figur 1A üben alle drei stempelförmigen Aktoren 1 eine Kraft auf den Wandkörper 4 des Fließkanals 5 aus, so dass die Querschnittsfläche Q des Fließkanals 5 in allen drei den Aktoren 1 zugeordneten Kanalabschnitten verringert ist, vorzugsweise so, dass die Kanalabschnitte verschlossen sind. Die Sensoranordnungen 6, 7, 8 detektieren somit keine oder nur eine geringe Menge an flüssigem Medium in den zugeordneten Kanalabschnitten.

In Figur 1B üben der erste Ventilstempel 2 und der Pumpenstempel 3 keine Kraft mehr auf den Wandkörper 4 aus, so dass der Fließkanal 5 in den zugehörigen Kanalabschnitten 11, 12 geöffnet ist und seine ursprüngliche Querschnittsfläche Q aufweist. Flüssiges Medium tritt nun in die beiden Kanalabschnitte 11, 12 ein. Der zweite Ventilstempel 2 übt weiterhin eine Kraft auf den Wandkörper 4 in dem dritten Kanalabschnitt 13 aus und verschließt den Fließkanal 5 in diesem Abschnitt 13. Die ersten zwei Sensoranordnungen 6 und 7 detektieren daher die Menge des in den ersten zwei Kanalabschnitten 11, 12 anwesenden flüssigen Mediums, die dritte Sensoranordnung 8 detektiert keine oder nur eine geringe Menge an flüssigem Medium in dem dritten Kanalabschnitt 13.

In Figur 1C üben beide Ventilstempel 2 eine Kraft auf den Wandkörper 4 aus, so dass der Fließkanal 5 in dem ersten Kanalabschnitt 11 und dem dritten Kanalabschnitt 13 zumindest weitgehend verschlossen ist. Der Pumpenstempel 3 übt weiterhin keine Kraft auf den Wandkörper 4 aus. Daher wird in dem zweiten Kanalabschnitt 12 durch die beiden verschlossenen Kanalabschnitte 11, 13 ein Volumensegment 14 eingegrenzt. Die zweite Sensoranordnung 7 detektiert die Menge des flüssigen Mediums in dem Volumensegment 14. Diese Menge ist unter anderem abhängig von den Abmessungen des zweiten Kanalabschnitts 12. Der als Wandkörper 4 vorzugsweise verwendete Schlauch kann zum Beispiel einen Innendurchmesser zwischen 0,1 und 0,3 mm aufweisen. Bei einer Länge des zweiten Kanalabschnitts 12 und damit auch des Volumensegments 14 zwischen 1 und 3 mm ergibt sich für das durch die Mikropumpe zu fördernde Volumensegment 14 ein Volumen von ca. 10 nl bis 200 nl. Die erste und die dritte Sensoranordnung 6, 8 detektieren kein oder wenig flüssiges Medium in dem ersten bzw. dritten Kanalabschnitt 11, 13.

In Figur 1D übt der zweite Ventilstempel 2 keine Kraft auf den Wandkörper 4 aus und der dritte Kanalabschnitt 13 ist geöffnet. Der erste Kanalabschnitt 11 bleibt weiterhin durch die Einwirkung des ersten Ventilstempels 2 verschlossen. Der Pumpenstempel 3 hat durch Kraftausübung auf den Wandkörper 4 den zweiten Kanalabschnitt 12 verschlossen und dadurch das flüssige Medium (aus dem Volumensegment 14) in den dritten Kanalabschnitt 13 und den sich daran anschließenden Fließkanal 5 verdrängt. Die erste und zweite Sensoranordnung 6, 7 detektieren kein oder wenig flüssiges Medium in dem ersten bzw. zweiten Kanalabschnitt 11, 12. Die dritte Sensoranordnung 8 detektiert eine Menge an flüssigem Medium in dem dritten Kanalabschnitt 13.

Anschließend beginnt die Bewegungssequenz wieder wie in Figur 1A dargestellt. Durch die drei Sensoranordnungen 6, 7, 8 kann das durch die drei Kanalabschnitte 11, 12, 13 transportierte flüssige Medium sicher überwacht werden.

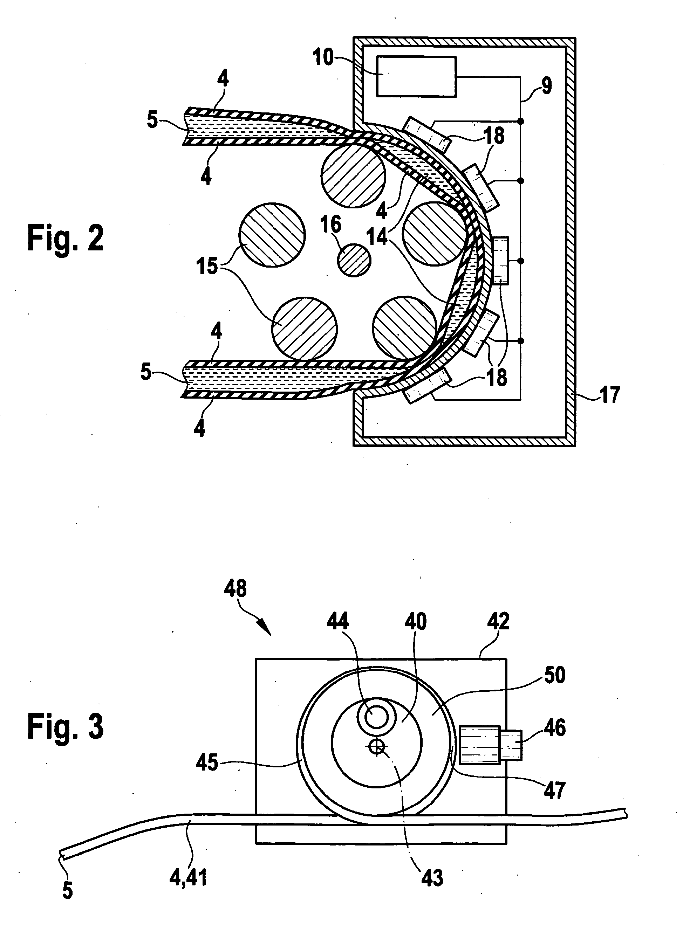

Figur 2 zeigt schematisch eine erfindungsgemäße Mikropumpe mit rollenförmigen Aktoren.

Die Mikropumpe weist fünf rollenförmige Aktoren 15 auf, die kreisförmig um eine rotierbare Welle 16 angeordnet sind. Ein Fließkanal 5 mit Wandkörper 4 verläuft bogenförmig entlang einer halbkreisförmigen Ausnehmung in einem Gehäuse 17. Der Wandkörper 4 ist zumindest im bogenförmigen Bereich aus einem elastischen Material gefertigt und lässt sich durch die rollenförmigen Aktoren 1 gegen das Gehäuse zusammendrücken, so dass seine innere Querschnittsfläche auf annähernd Null verringert werden kann. Außerhalb des Wandkörpers 4 sind an fünf Kanalabschnitten Sensoranordnungen 18 angeordnet, die über Signalleitungen 9 mit einer Auswerteeinrichtung 10 verbunden sind. Zwei nebeneinander liegende rollenförmige Aktoren 15 grenzen ein Volumensegment 14 ein, wenn beide gleichzeitig den Fließkanal 5 durch Kraftausübung auf den Wandkörper 4 verschließen. Beim Rotieren der rotierenden Welle 16 rotieren die rollenförmigen Aktoren 15 um die Welle 16 und bewegen sich dadurch entlang des bogenförmigen Fließkanals 5, so dass die zwischen zwei rollenförmigen Aktoren 15 eingegrenzten Volumensegmente 14 in dem Fließkanal 5 weiter transportiert werden. Auf diesem Prinzip beruht die Pumpwirkung der Rotationspumpe. Die Sensoranordnungen detektieren die Menge an flüssigem Medium in einem an sie angrenzenden Kanalabschnitt. Bei der Rotation der Welle 16 und resultierenden Bewegung der rollenförmigen Aktoren 15 nimmt die Menge an detektiertem flüssigem Medium neben jedem der Sensoranordnungen 18 nacheinander periodisch zu und ab. Bei einer Fehlfunktion der Pumpe oder einer Okklusion ergeben sich davon abweichende detektierte Signale.

Figur 3 zeigt schematisch eine erfindungsgemäße Mikropumpe mit scheibenförmigem Aktor.

Bei dieser Ausführungsform der Erfindung dient eine rotierende erste Scheibe 40 als Aktor. Als Wandkörper 4, der den Fließkanal 5 enthält, dient ein Schlauch 41. In dem an die erste Scheibe 40 angrenzenden Bereich innerhalb eines Gehäuses 42 befindet sich eine zweite Scheibe 50, an der der Fließkanal 5 annähernd ringförmig anliegt. Die Scheibe 40 dreht sich zum Pumpen von flüssigem Medium um die exzentrische Achse 43. Auf der ersten Scheibe 40 ist eine Rolle 44 angeordnet, die beim Rotieren der ersten Scheibe 40 um die Achse 43 umlaufend an eine zweite Scheibe 50 gedrückt wird, die umlaufend an den ringförmig verlaufenden Wandkörper 45 des Fließkanals 5 angedrückt wird, wodurch die Querschnittsfläche des elastisch ausgebildeten, ringförmig verlaufenden Wandkörpers 45 umlaufend verringert und dadurch eine Förderung des flüssigen Mediums erreicht wird. Zum Antrieb der ersten Scheibe 40 sind in dem Gehäuse 42 vorzugsweise ein Mikromotor mit üblichem Getriebe oder ein Mikromotor mit Spindel (nicht dargestellt) enthalten. Ferner ist in dem Gehäuse 42 außerhalb des Wandkörpers 45 an einem Kanalabschnitt 47 eine Sensoranordnung 46 angeordnet, die eine Menge des flüssigen Mediums in dem Kanalabschnitt 47 detektieren kann. Durch das mechanische Prinzip des Exzenters wird eine hohe Zuverlässigkeit der Mikropumpe erreicht. Freier, unkontrollierter Fluss des flüssigen Mediums ist nicht möglich. Durch die um die exzentrische Achse 43 laufende erste Scheibe 40 und die dadurch bewirkte konstante Förderung ist die Förderung von der (Dreh-)Lage der ersten Scheibe 40 nahezu unabhängig. Daher führt bei dieser Exzenterpumpe 48 die Überwachung durch eine Sensoranordnung 46 zu guter Qualitätssicherung der Pumpenfunktion. Es ist jedoch auch eine Überwachung mittels mehrerer Sensoranordnungen möglich. Die Exzenterpumpe 48 (zum Beispiel mit Spindelantrieb) kann sehr klein gebaut werden, so dass sie gut in ein Gehäuse zum Beispiel mit einer Infusionsnadelanordnung 37 einer Vorrichtung zur dosierten Verabreichung eines flüssigen Medikaments integriert werden kann, ohne dass der Tragekomfort der Infusionsnadelanordnung 37 für einen Patienten wesentlich beeinträchtigt wird bei gleichzeitiger Erhöhung der Gesamtzuverlässigkeit.

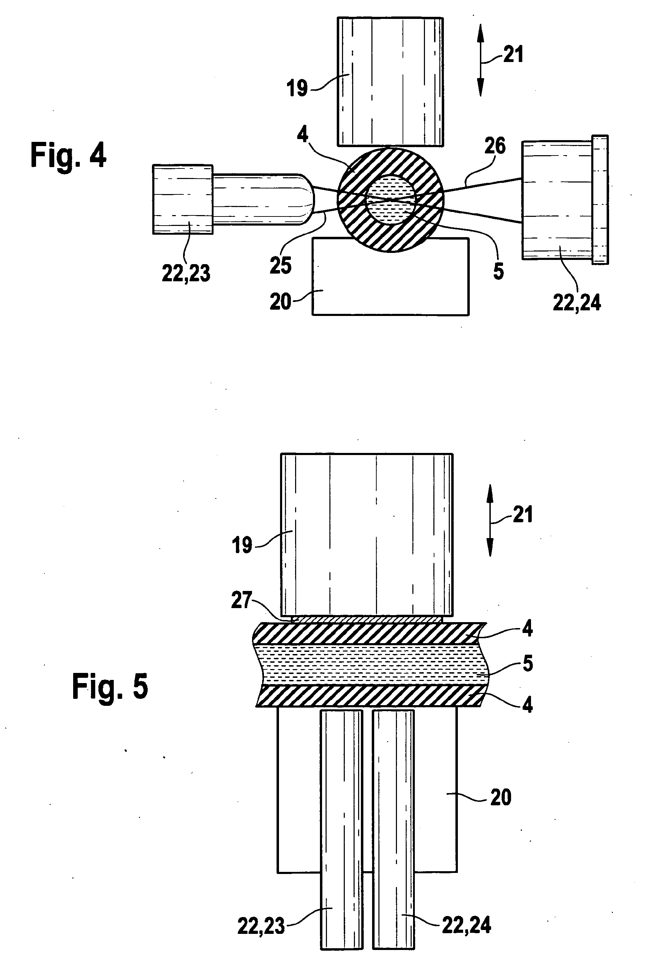

Figur 4 zeigt schematisch das Messprinzip einer ersten Sensoranordnung zur Messung der Absorption oder Fluoreszenz von elektromagnetischer Strahlung in einer erfindungsgemäßen Mikropumpe.

An dem Wandkörper 4 eines Fließkanals 5 ist ein Aktor 19 (zum Beispiel ein Stempel oder Exzenter) und gegenüberliegend ein Widerlager 20 angeordnet. Der Aktor 19 ist in der angezeigten Bewegungsrichtung 21 beweglich und kann dabei eine Kraft auf den Wandkörper 4 zur Änderung der Querschnittsfläche des Fließkanals 5 ausüben. Eine Sensoranordnung 22 umfasst ein elektromagnetische Strahlung emittierendes Element 23 (zum Beispiel eine LED) und ein an dem Wandkörper 4 gegenüberliegend angeordnetes, elektromagnetische Strahlung detektierendes Element 24 (zum Beispiel eine Photodiode oder das Ende eines Lichtleiters, der zu einer Photodiode führt). Gegebenenfalls enthält das detektierende Element 24 ein (nicht dargestelltes) Filterelement, zum Beispiel bei Fluoreszenzmessungen oder um Umgebungsstrahlung abzuschwächen. Das emittierende Element 23 ist auf den Wandkörper 4 gerichtet, so dass die emittierte elektromagnetische Strahlung 25 den Wandkörper 4 auf einer Seite, den Fließkanal 5 und den Wandkörper 4 auf der anderen Seite durchläuft. In dem Fließkanal 5 wird die emittierte elektromagnetische Strahlung 25 je nach darin enthaltener Menge des flüssigen Mediums absorbiert bzw. erzeugt Fluoreszenz. Der verbleibende nicht absorbierte bzw. der fluoreszierende Anteil der Strahlung tritt aus dem Wandkörper 4 aus (transmittierte elektromagnetische Strahlung 26) und kann von dem detektierenden Element 24 detektiert und von einer Auswerteeinrichtung ausgewertet werden.

Figur 5 zeigt schematisch eine zweite Sensoranordnung zur Messung der Absorption oder Fluoreszenz von elektromagnetischer Strahlung oder der Reflexion von Ultraschall in einer erfindungsgemäßen Mikropumpe.

An dem Wandkörper 4 eines Fließkanals 5 ist ein Aktor 19 und gegenüberliegend ein Widerlager 20 angeordnet. Der Aktor 19 (zum Beispiel ein Stempel, eine Rolle oder ein Exzenter) ist in der angezeigten Bewegungsrichtung 21 beweglich und kann dabei eine Kraft auf den Wandkörper 4 zur Änderung der Querschnittsfläche des Fließkanals 5 ausüben.

Eine Sensoranordnung 22 umfasst in einer ersten Ausführungsform ein elektromagnetische Strahlung emittierendes Element 23 (zum Beispiel eine LED) und ein an dem Wandkörper 4 daneben angeordnetes elektromagnetische Strahlung detektierendes Element 24 (zum Beispiel eine Photodiode oder einen Lichtwellenleiter, gegebenenfalls mit Filter). Der Wandkörper 4 weist einen Wandbereich 27 auf, der (zumindest teilweise) reflektierend ausgebildet ist (zum Beispiel eine Licht reflektierende Schicht). Die von dem emittierenden Element 23 emittierte elektromagnetische Strahlung (nicht dargestellt) durchläuft den Wandkörper 4, den Fließkanal 5 und trifft auf den reflektierenden Wandbereich 27, von wo aus ein Teil der Strahlung in Richtung des detektierenden Elements 24 reflektiert wird. In dem Fließkanal 5 wird die elektromagnetische Strahlung je nach darin enthaltener Menge des flüssigen Mediums absorbiert bzw. erzeugt Fluoreszenz. Der verbleibende, nicht absorbierte bzw. der fluoreszierende Anteil der Strahlung tritt aus dem Wandkörper 4 aus und kann von dem detektierenden Element 24 detektiert und von einer Auswerteeinheit ausgewertet werden.

Die Sensoranordnung 22 umfasst in einer zweiten Ausführungsform einen Ultraschallsender 23 (basierend zum Beispiel auf einem piezolektrischen Element) und einen an dem Wandkörper 4 daneben angeordneten Ultraschallempfänger 24. Der Wandkörper 4 enthält zumindest in einem an den dargestellten Kanalabschnitt angrenzenden Bereich ein Material mit einer bestimmten akustischen Impedanz, die im Hinblick auf die akustische Impedanz des flüssigen Mediums gewählt wird. Der von dem Ultraschallsender 23 emittierte Ultraschall durchläuft den Wandkörper 4, den Fließkanal 5 und wird in Abhängigkeit von der akustischen Impedanz-Fehlanpassung zwischen dem in dem Fließkanal 5 enthaltenen Material und dem Wandkörpermaterial in Richtung des Ultraschallempfängers 24 reflektiert. Der reflektierte Anteil des Ultraschalls tritt aus dem Wandkörper 4 aus und kann von dem Ultraschallempfänger 24 detektiert und von einer Auswerteeinheit zur Bestimmung der Menge an flüssigem Medium in dem Kanalabschnitt ausgewertet werden.

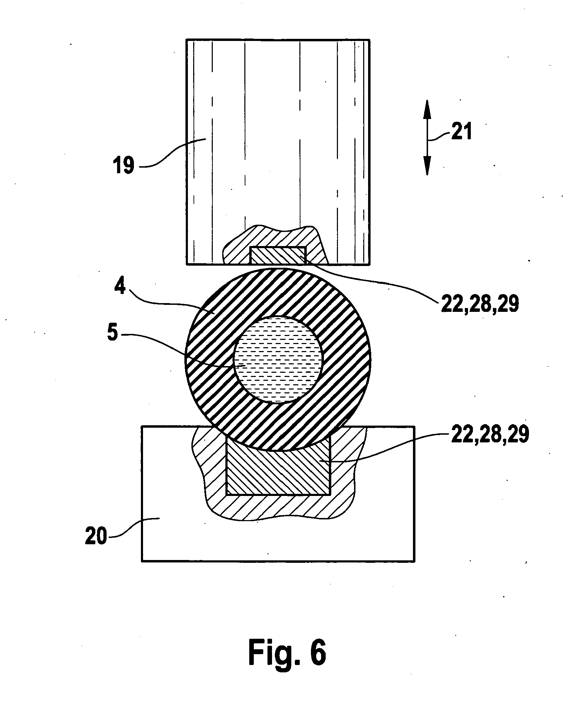

Figur 6 zeigt schematisch eine Sensoranordnung zur Kapazitätsmessung oder zur Ultraschallmessung in einer erfindungsgemäßen Mikropumpe.

An dem Wandkörper 4 eines Fließkanals 5 ist ein Aktor 19 und gegenüberliegend ein Widerlager 20 angeordnet. Der Aktor 19 ist in der angezeigten Bewegungsrichtung 21 beweglich und kann dabei eine Kraft auf den Wandkörper 4 zur Änderung der Querschnittsfläche des Fließkanals 5 ausüben.

Gemäß einer ersten Ausführungsform umfasst eine Sensoranordnung 22 einen Kondensator 28, der auf zwei Seiten eines Kanalabschnitts des Fließkanals 5 außerhalb an dem Wandkörper 4 angeordnete Kondensatorplatten 29 enthält. Die Kapazität des Kondensators 28 hängt unter anderem von der Menge an in dem Fließkanal 5 zwischen den Kondensatorplatten 29 enthaltenem flüssigem Medium ab. Eine (nicht dargestellte) Messanordnung misst die Kapazität des Kondensators 28 zur Bestimmung dieser Menge.

Gemäß einer zweiten Ausführungsform umfasst eine Sensoranordnung 22 einen Ultraschallsender 29 und einen Ultraschallempfänger 29, die auf zwei gegenüberliegenden Seiten eines Kanalabschnitts des Fließkanals 5 außerhalb an dem Wandkörper 4 angeordnet sind. Der transmittierte Anteil des von dem Ultraschallsender 29 zu dem Ultraschallempfänger 29 durch den Fließkanal 5 transmittierten Ultraschalls hängt unter anderem von der Menge an in dem Fließkanal 5 zwischen Ultraschallsender 29 und Ultraschallempfänger 29 enthaltenem flüssigem Medium ab.

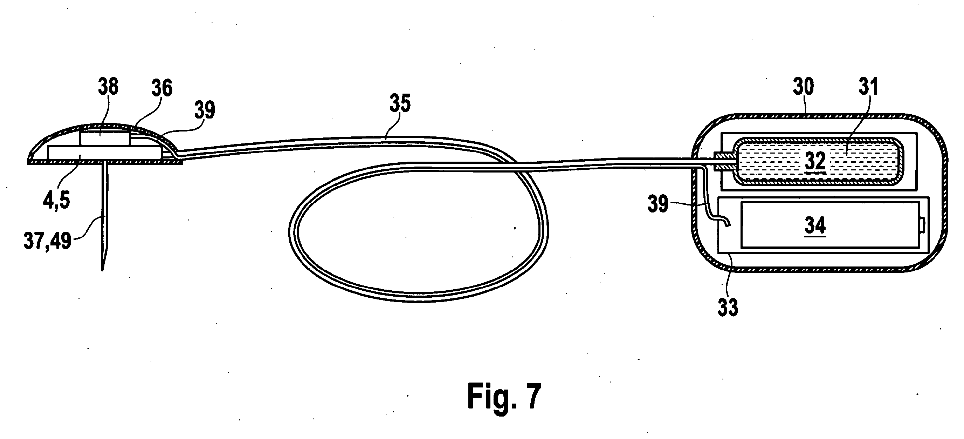

Figur 7 zeigt schematisch eine erfindungsgemäße Vorrichtung zur dosierten Verabreichung eines flüssigen Medikaments.

Die Vorrichtung umfasst ein in einem ersten Gehäuse 30 angeordnetes Reservoir 31 mit flüssigem Medikament 32. Dabei handelt es sich zum Beispiel um einen Insulinbehälter, der als Blisterverpackung ausgeführt ist, die sich beim Entleeren zusammenfaltet. In dem ersten Gehäuse 30 befindet sich ferner eine Steuereinrichtung 33 mit Batterie 34. An die Steuereinrichtung 33 können beispielsweise per manuellem Bedienelement (nicht dargestellt) oder ganz oder teilweise durch eine Fernbedieneinheit (nicht dargestellt) Vorgaben durch einen Benutzer übermittelt werden. An das Reservoir 31 ist ein Verbindungsschlauch 35 angeschlossen. Der Verbindungsschlauch 35 transportiert flüssiges Medikament 32 aus dem Reservoir 31 zu einem zweiten Gehäuse 36. Entlang des Verbindungsschlauchs 35 verlaufen ferner Leitungen 39, die zur elektrischen Versorgung und Signalleitung vorgesehen und mit der Steuereinrichtung 33 verbunden sind. In dem zweiten Gehäuse 36 befindet sich eine Infusionsnadelanordnung 37, die über einen Fließkanal 5 in einem Wandkörper 4 mit dem Verbindungsschlauch 35 und dem Reservoir 31 verbunden ist. Die Infusionsnadelanordnung 37 enthält eine Infusionsnadel 49, die sich gegebenenfalls "schwimmend" in dem zweiten Gehäuse 36 bewegen kann, so dass Bewegungen des Patienten und die dadurch ausgelösten Relativbewegungen von Infusionsnadel 49 und Gehäuse 36 nicht zu Schmerzen führen. In dem zweiten Gehäuse 36 ist ferner eine erfindungsgemäße Mikropumpe 38 angeordnet, die auf den Wandkörper 4 des Fließkanals zum Pumpen des flüssigen Medikaments 32 einwirkt. Die Mikropumpe 38 wird über die Leitungen 39 mit Energie und Steuersignalen versorgt und übermittelt Messsignale der in der Mikropumpe 38 enthaltenen Sensoranordnungen (nicht dargestellt) an die Steuereinrichtung 33. Der Wandkörper 4 des Fließkanals 5 ist vorzugsweise in oder an der Mikropumpe lösbar montiert, so dass man die Mikropumpe ohne Fließkanal 5 aus dem zweiten Gehäuse 36 entfernen und den Rest der erfindungsgemäßen Vorrichtung entsorgen kann. Die Mikropumpe kann wieder verwendet werden, zum Beispiel indem ein neuer Wandkörper 4 eines Fließkanals 5 an/in die Mikropumpe eingeklipst wird, um eine neue erfindungsgemäße Vorrichtung zur dosierten Verabreichung des flüssigen Medikaments 32 zu erhalten.

### Bezugszeichenliste

- 1: stempelförmige Aktoren
- 2: Ventilstempel
- 3: Pumpenstempel
- 4: Wandkörper
- 5: Fließkanal
- 6: erste Sensoranordnung
- 7: zweite Sensoranordnung
- 8: dritte Sensoranordnung
- 9: Signalleitungen
- 10: Auswerteeinrichtung
- 11: erster Kanalabschnitt
- 12: zweiter Kanalabschnitt
- 13: dritter Kanalabschnitt
- 14: Volumensegment
- 15: rollenförmige Aktoren
- 16: rotierbare Welle
- 17: Gehäuse
- 18: Sensoranordnungen
- 19: Aktor
- 20: Widerlager
- 21: Bewegungsrichtung
- 22: Sensoranordnung
- 23: elektromagnetische Strahlung emittierendes Element oder Ultraschallsender
- 24: elektromagnetische Strahlung detektierendes Element oder Ultraschallempfänger
- 25: emittierte elektromagnetische Strahlung
- 26: transmittierte elektromagnetische Strahlung
- 27: Wandbereich
- 28: Kondensator
- 29: Kondensatorplatten, Ultraschallsender, Ultraschallempfänger
- 30: erstes Gehäuse
- 31: Reservoir
- 32: flüssiges Medikament
- 33: Steuereinrichtung
- 34: Batterie
- 35: Verbindungsschlauch
- 36: zweites Gehäuse
- 37: Infusionsnadelanordnung
- 38: Mikropumpe
- 39: Leitungen
- 40: erste Scheibe
- 41: Schlauch
- 42: Gehäuse
- 43: exzentrische Achse
- 44: Rolle
- 45: ringförmig verlaufender Wandkörper
- 46: Sensoranordnung
- 47: Kanalabschnitt
- 48: Exzenterpumpe
- 49: Infusionsnadel
- 50: zweite Scheibe

## Patentansprüche

1. Mikropumpe zum peristaltischen Pumpen eines flüssigen Mediums, umfassend
a) mindestens einen Fließkanal (5) mit einem Wandkörper (4), wobei der Fließkanal (5) eine Querschnittsfläche aufweist, die zumindest in Teilabschnitten des Fließkanals (5) durch Krafteinwirkung auf den Wandkörper (4) des Fließkanals (5) veränderbar ist und
b) Aktoren (1,15,19), die so angeordnet ist, dass sie auf den Wandkörper (4) des Fließkanals (5) in einem Kanalabschnitt (11, 12, 13) eine Kraft zum Verringern der Querschnittsfläche in dem Kanalabschnitt (11, 12, 13) ausüben können,
wobei die Mikropumpe mindestens eine Sensoranordnung (6, 7 ,8, 18, 22) zur Detektion einer Menge des flüssigen Mediums in dem Kanalabschnitt (11, 12, 13) aufweist, wobei die Sensoranordnung (6, 7, 8, 18, 22) außerhalb des Fließkanals (5) angeordnet ist, wobei die Sensoranordnung (6, 7 ,8, 18, 22) ein Messsignal detektiert, welches unter Kenntnis anderer Parameter die Berechnung eines Volumens des flüssigen Mediums in dem Kanalabschnitt ermöglicht, wobei mindestens zwei der Aktoren so angeordnet sind, dass sie auf den Wandkörper (4) des Fließkanals (5) in mindestens zwei Kanalabschnitten eine Kraft zum vorübergehenden Verschließen des jeweiligen Kanalabschnitts ausüben können, so dass ein Volumensegment (14) in dem Fließkanal (5) eingrenzbar ist, wobei eine Sensoranordnung (6, 7, 8, 18, 22) außerhalb des Fließkanals (5) zur Detektion der Menge des flüssigen Mediums in dem Volumensegment (14) vorgesehen ist, und **gekennzeichnet dadurch, dass** mindestens zwei weitere Sensoranordnungen (6, 7, 8, 18, 22) zur Detektion der Menge des flüssigen Mediums in den mindestens zwei verschließbaren Kanalabschnitten vorgesehen sind, wobei die mindestens zwei weiteren Sensoranordnungen außerhalb des Fließkanals (5) angeordnet sind.

2. Mikropumpe gemäß Anspruch 1, **gekennzeichnet durch** mindestens drei nebeneinander außerhalb des Fließkanals (5) angeordnete stempelförmige Aktoren (1), die mindestens zwei Ventilstempel (2) und mindestens einen Pumpenstempel (3) umfassen.

3. Mikropumpe gemäß Anspruch 1, **dadurch gekennzeichnet, dass** die Mikropumpe eine Rotationspumpe mit rollenförmigen Aktoren (15) ist, wobei die rollenförmigen Aktoren (15) kreisförmig um eine rotierbare Welle (16) angeordnet sind und der Fließkanal (5) bogenförmig entlang eines an die rollenförmigen Aktoren (15) angrenzenden Bereichs verläuft.

4. Mikropumpe gemäß Anspruch 1, **dadurch gekennzeichnet, dass** als Aktor eine rotierbare Scheibe (40) dient, die um eine exzentrische Achse (43) rotiert, wobei der Fließkanal (5) annähernd ringförmig in einem an die rotierbare Scheibe (40) oder an eine durch die rotierbare Scheibe (40) betätigbare weitere Scheibe (50) angrenzenden Bereich verläuft.

5. Mikropumpe gemäß einem der Ansprüche 1 bis 4, **dadurch gekennzeichnet, dass** die Sensoranordnung (6, 7, 8, 18, 22) ein elektromagnetische Strahlung emittierendes Element (23) umfasst, das auf einen zumindest teilweise strahlungsdurchlässigen Bereich des Wandkörpers (4) des Fließkanals (5) gerichtet ist, und dass die Sensoranordnung (6, 7, 8, 18, 22) ein elektromagnetische Strahlung detektierendes Element (24) umfasst, das ebenfalls auf einen zumindest teilweise strahlungsdurchlässigen Bereich des Wandkörpers (4) des Fließkanals (5) gerichtet ist und das so angeordnet ist, dass es aus dem Bereich des Wandkörpers (4) austretende elektromagnetische Strahlung, die den Fließkanal (5) durchquert hat, detektieren kann.

6. Mikropumpe gemäß der Anspruch 5, **dadurch gekennzeichnet, dass** der Wandkörper (4) einen Wandbereich (27) aufweist, der zumindest teilweise reflektierend ausgebildet ist und dass das elektromagnetische Strahlung detektierende Element (24) so angeordnet ist, dass es von dem elektromagnetische Strahlung emittierenden Element (23) emittierte und von dem Wandbereich (27) reflektierte Strahlung detektieren kann.

7. Mikropumpe gemäß einem der Ansprüche 1 bis 4, **dadurch gekennzeichnet, dass** die Sensoranordnung (6, 7, 8, 18, 22) einen Kondensator (28) umfasst, der auf zwei Seiten des Kanalabschnitts des Fließkanals (5) außerhalb des Wandkörpers (4) angeordnete Kondensatorplatten (29) enthält und dass die Sensoranordnung (6, 7, 8, 18, 22) eine Messanordnung zur Messung der Kapazität des Kondensators (28) umfasst.

8. Mikropumpe gemäß einem der Ansprüche 1 bis 4, **dadurch gekennzeichnet, dass** die Sensoranordnung (6, 7, 8, 18, 22) einen Ultraschallsensor umfasst, der einen Ultraschallsender und einen Ultraschallempfänger enthält, die auf zwei Seiten des Kanalabschnitts des Fließkanals (5) außerhalb des Wandkörpers (4) angeordnet sind.

9. Vorrichtung zur dosierten Verabreichung eines flüssigen Medikaments (32), enthaltend
i) eine Mikropumpe (38) gemäß einem der Ansprüche 1 bis 8,
ii) ein Reservoir (31) mit dem flüssigen Medikament (32) und
iii) eine Infusionsnadelanordnung (37),
wobei das Reservoir (31) mit der Infusionsnadelanordnung (37) über den Fließkanal (5) verbunden ist.

10. Vorrichtung gemäß Anspruch 9, **dadurch gekennzeichnet, dass** der Wandkörper (4) des Fließkanals (5) in der Mikropumpe (38) lösbar montiert ist.

11. Vorrichtung gemäß einem der Ansprüche 9 oder 10, **dadurch gekennzeichnet, dass** die Mikropumpe (38) in einem gemeinsamen Gehäuse mit dem Reservoir (31) oder mit der Infusionsnadelanordnung (37) oder separat außerhalb des Wandkörpers (4) des Fließkanals (5) zwischen dem Reservoir (31) und der Infusionsnadelanordnung (37) angeordnet ist.

## Claims

1. Micropump for the peristaltic pumping of a liquid medium, comprising
a) at least one flow channel (5) with a wall body (4), wherein the flow channel (5) has a cross-sectional area which can be changed at least in subsections of the flow channel (5) through the action of force on the wall body (4) of the flow channel (5) and
b) actuators (1, 15, 19) which are arranged so that they can exert a force on the wall body (4) of the flow channel (5) in a channel section (11, 12, 13) for reducing the cross-sectional area in the channel section (11, 12, 13),
wherein the micropump has at least one sensor arrangement (6, 7, 8, 18, 22) for detecting a quantity of the liquid medium in the channel section (11, 12, 13), wherein the sensor arrangement (6, 7, 8, 18, 22) is arranged outside of the flow channel (5), wherein the sensor arrangement (6, 7, 8, 18, 22) detects a measurement signal which, having knowledge of other parameters, enables a volume of the liquid medium in the channel section to be calculated,
wherein
at least two of the actuators are arranged so that they can exert a force on the wall body (4) of the flow channel (5) in at least two channel sections for temporarily closing the respective channel section, so that a volumetric segment (14) in the flow channel (5) can be delimited,
wherein one sensor arrangement (6, 7, 8, 18, 22) is provided outside of the flow channel (5) for detecting the quantity of the liquid medium in the volumetric segment (14), and **characterised in that** at least two further sensor arrangements (6, 7, 8, 18, 22) are provided for detecting the quantity of the liquid medium in the at least two closable channel sections, wherein the at least two further sensor arrangements (6, 7, 8, 18, 22) are arranged outside of the flow channel (5).

2. Micropump according to Claim 1, **characterised by** at least three plunger-shaped actuators (1) which are arranged side by side outside of the flow channel (5) and comprise at least two valve plungers (2) and at least one pump plunger (3).

3. Micropump according to Claim 1, **characterised in that** the micropump is a rotary pump with roll-shaped actuators (15), wherein the roll-shaped actuators (15) are arranged in the shape of a circle about a rotatable shaft (16) and the flow channel (5) extends in the shape of an arc along a region which is adjacent to the roll-shaped actuators (15).

4. Micropump according to Claim 1, **characterised in that** a rotatable disc (40) serves as actuator, which disc rotates about an eccentric axis (43), wherein the flow channel (5) extends approximately in the shape of a ring in a region which is adjacent to the rotatable disc (40) or to a further disc (50) which can be actuated by the rotatable disc (40).

5. Micropump according to any one of Claims 1 to 4, **characterised in that** the sensor arrangement (6, 7, 8, 18, 22) comprises an element (23) which emits electromagnetic radiation and is directed at a region of the wall body (4) of the flow channel (5) which is at least partly radiation-permeable, and that the sensor arrangement (6, 7, 8, 18, 22) comprises an element (24) which detects electromagnetic radiation, is likewise directed at a region of the wall body (4) of the flow channel (5) which is at least partly radiation-permeable and is arranged so that it can detect electromagnetic radiation having emerged from the region of the wall body (4) and passed through the flow channel (5).

6. Micropump according to Claim 5, **characterised in that** the wall body (4) has a wall region (27) which is formed so as to be at least partly reflective, and that the element (24) detecting electromagnetic radiation is arranged so that it can detect radiation emitted by the element (23) emitting electromagnetic radiation and reflected by the wall region (27).

7. Micropump according to any one of Claims 1 to 4, **characterised in that** the sensor arrangement (6, 7, 8, 18, 22) comprises a capacitor (28) which includes capacitor plates (29) arranged on two sides of the channel section of the flow channel (5) outside of the wall body (4), and that the sensor arrangement (6, 7, 8, 18, 22) comprises a measurement arrangement for measuring the capacitance of the capacitor (28).

8. Micropump according to any one of Claims 1 to 4, **characterised in that** the sensor arrangement (6, 7, 8, 18, 22) comprises an ultrasonic sensor which includes an ultrasonic transmitter and an ultrasonic receiver which are arranged on two sides of the channel section of the flow channel (5) outside of the wall body (4).

9. Device for the metered administration of a liquid medication (32), including
i) a micropump (38) according to any one of Claims 1 to 8,
ii) a reservoir (31) with the liquid medication (32) and
iii) an infusion needle arrangement (37),
wherein the reservoir (31) is connected to the infusion needle arrangement (37) by way of the flow channel (5).

10. Device according to Claim 9, **characterised in that** the wall body (4) of the flow channel (5) is mounted in the micropump (38) in a releasable manner.

11. Device according to either of Claims 9 and 10, **characterised in that** the micropump (38) is arranged in a common casing with the reservoir (31) or with the infusion needle arrangement (37) or separately outside of the wall body (4) of the flow channel (5) between the reservoir (31) and the infusion needle arrangement (37).

## Revendications

1. Micro-pompe péristaltique destinée au pompage d'un milieu liquide, comprenant
a) au moins un canal d'écoulement (5) muni d'un corps de paroi (4), le canal d'écoulement (5) présentant une surface de section transversale pouvant varier, au moins dans des portions du canal d'écoulement (5), sous l'effet d'une force appliquée sur le corps de paroi (4) du canal d'écoulement (5), et
b) des actionneurs (1, 15, 19) disposés de telle sorte qu'ils puissent exercer sur le corps de paroi (4) du canal d'écoulement (5), dans une portion de canal (11, 12, 13), une force destinée à diminuer la surface de section transversale dans la portion de canal (11, 12, 13),
ladite micro-pompe comportant au moins un ensemble capteur (6, 7, 8, 18, 22) destiné à détecter une quantité du milieu liquide dans la portion de canal (11, 12, 13), ledit ensemble capteur (6, 7, 8, 18, 22) étant disposé à l'extérieur du canal d'écoulement (5), ledit ensemble capteur (6, 7, 8, 18, 22) détectant un signal de mesure permettant, en connaissance d'autres paramètres, de calculer un volume du milieu liquide dans ladite portion de canal, au moins deux desdits actionneurs étant disposés de telle sorte qu'ils puissent exercer sur le corps de paroi (4) du canal d'écoulement (5), dans au moins deux portions de canal, une force destinée à obturer temporairement la portion de canal respective de manière à pouvoir confiner un segment volumétrique (14) dans le canal d'écoulement (5), un ensemble capteur (6, 7, 8, 18, 22) étant prévu à l'extérieur du canal d'écoulement (5) pour détecter la quantité du milieu liquide dans ledit segment volumétrique (14), et **caractérisée en ce qu'**au moins deux autres ensembles capteurs (6, 7, 8, 18, 22) sont prévus pour détecter la quantité du milieu liquide dans lesdites au moins deux portions de canal obturables, lesdits au moins deux autres ensembles capteurs étant disposés à l'extérieur du canal d'écoulement (5).

2. Micro-pompe selon la revendication 1, **caractérisée par** au moins trois actionneurs (1) en forme de piston, disposés côte à côte à l'extérieur du canal d'écoulement (5), et qui comprennent au moins deux pistons de soupape (2) et au moins un piston de pompe (3).

3. Micro-pompe selon la revendication 1, **caractérisée en ce que** la micro-pompe est une pompe rotative dotée d'actionneurs (15) en forme de galet, les actionneurs (15) en forme de galet étant disposés en forme de cercle autour d'un arbre rotatif (16), et le canal d'écoulement (5) s'étendant en forme d'arc le long d'une zone contiguë aux actionneurs (15) en forme de galet.

4. Micro-pompe selon la revendication 1, **caractérisée en ce qu'**un disque rotatif (40) est utilisé comme actionneur, lequel tourne autour d'un axe excentré (43), le canal d'écoulement (5) s'étendant approximativement en forme d'anneau dans une zone contiguë audit disque rotatif (40) ou à un disque supplémentaire (50) actionnable par le disque rotatif (40).

5. Micro-pompe selon l'une des revendications 1 à 4, **caractérisée en ce que** l'ensemble capteur (6, 7, 8, 18, 22) comprend un élément émetteur de rayonnement électromagnétique (23), lequel est dirigé sur une zone du corps de paroi (4) du canal d'écoulement (5), zone qui est au moins partiellement perméable au rayonnement, et **en ce que** l'ensemble capteur (6, 7, 8, 18, 22) comprend un élément détecteur de rayonnement électromagnétique (24), lequel est également dirigé sur une zone du corps de paroi (4) du canal d'écoulement (5), zone qui est au moins partiellement perméable au rayonnement, et lequel est disposé de telle sorte qu'il puisse détecter le rayonnement électromagnétique sortant de la zone du corps de paroi (4) après avoir traversé le canal d'écoulement (5).

6. Micro-pompe selon la revendication 5, **caractérisée en ce que** le corps de paroi (4) comporte une zone de paroi (27), laquelle est au moins partiellement réfléchissante, et **en ce que** l'élément détecteur de rayonnement magnétique (24) est disposé de telle sorte qu'il puisse détecter le rayonnement émis par l'élément émetteur de rayonnement (23) et réfléchi par la zone de paroi (27).

7. Micro-pompe selon l'une des revendications 1 à 4, **caractérisée en ce que** l'ensemble capteur (6, 7, 8, 18, 22) comprend un condensateur (28), lequel comporte des plaques de condensateur (29) disposées sur deux côtés de la portion de canal du canal d'écoulement (5), à l'extérieur du corps de paroi (4), et **en ce que** l'ensemble capteur (6, 7, 8, 18, 22) comporte un ensemble de mesure pour mesurer la capacité du condensateur (28).

8. Micro-pompe selon l'une des revendications 1 à 4, **caractérisée en ce que** l'ensemble capteur (6, 7, 8, 18, 22) comprend un capteur à ultrasons, lequel comporte un émetteur d'ultrasons et un récepteur d'ultrasons disposés sur deux côtés de la portion de canal du canal d'écoulement (5), à l'extérieur du corps de paroi (4).

9. Dispositif destiné à l'administration dosée d'un médicament liquide (32), comprenant
i) une micro-pompe (38) selon l'une des revendications 1 à 8,
ii) un réservoir (31) contenant le médicament liquide (32), et
iii) un ensemble de perfusion (37),
le réservoir (31) étant relié à l'ensemble formant aiguille de perfusion (37) par l'intermédiaire du canal d'écoulement (5).

10. Dispositif selon la revendication 9, **caractérisé en ce que** le corps de paroi (4) du canal d'écoulement (5) est monté de manière amovible dans la micro-pompe (38).

11. Dispositif selon l'une des revendications 9 ou 10, **caractérisé en ce que** la micro-pompe (38) est placée dans un boitier commun avec le réservoir (31) ou avec l'ensemble aiguille de perfusion (37), ou bien séparément à l'extérieur du corps de paroi (4) du canal d'écoulement (5), entre le réservoir (31) et l'ensemble formant de perfusion (37).
